# EUROPÄISCHE PATENTSCHRIFT

(11) **EP 0 839 574 B1**
(45) Veröffentlichungstag und Bekanntmachung des Hinweises auf die Patenterteilung: **30.05.2001**
(21) Anmeldenummer: 97118631.7
(22) Anmeldetag: 27.10.1997
(51) Int. Cl.: B01J 23/89, C07C 209/16, C07C 209/26

(54) **Katalysatoren für die Aminierung von Alkylenoxiden, Alkoholen, Aldehyden und Ketonen**
Catalysts for the amination of alkylene oyxdes, alcohols, aldehydes and ketones
Catalyseurs pour l'amination d'oxydes d'alkylène, alcools, aldéhydes et cétones

(30) Priorität: 31.10.1996 DE 19644107
(43) Veröffentlichungstag der Anmeldung: 06.05.1998
(73) Patentinhaber: BASF AKTIENGESELLSCHAFT, 67056 Ludwigshafen (DE)
(72) Erfinder: Wulff-Döring, Joachim, Dr., 67227 Frankenthal (DE); Melder, Johann-Peter, Dr., 67141 Neuhofen (DE); Schulz, Gerhard, Dr,., 67069 Ludwigshafen (DE); Voit, Guido, Dr., 69198 Schriesheim (DE); Gutschoven, Frank, 3600 Gent (BE); Harder, Wolfgang, Dr., 69469 Weinheim (DE)
(74) Vertreter: Isenbruck, Günter, Dr.

(56) Entgegenhaltungen:
- EP-A- 0 146 508
- EP-A- 0 254 335
- EP-A- 0 312 253
- EP-A- 0 356 046
- DE-A- 3 523 074

## Beschreibung

Die Erfindung betrifft Ruthenium, Nickel und/oder Kobalt enthaltende Katalysatoren, die zur Aminierung von Alkylenoxiden, Alkoholen, Aldehyden und Ketonen verwendet werden können. Weiterhin betrifft die Erfindung Verfahren zur Herstellung der Katalysatoren, deren Verwendung in Aminierungsreaktionen und Verfahren zur Herstellung von Aminierungsprodukten.

Aus der EP-A-0 146 508 sind Dehydrierungs-/Hydrierungskatalysatoren bekannt, die Ruthenium, Nickel und/oder Kobalt enthalten. Die verwendeten Katalysatoren enthalten ca. 0,25 - 1,0 Gew.-% Ruthenium und 7,5 oder 10 Gew.-% Nickel oder 10 Gew.-% Kobalt oder 4 Gew.-% Nickel und 4 Gew.-% Kobalt, bezogen auf das Gesamtgewicht des Katalysators, auf einem Aluminiumoxidträger. Die Katalysatoren können neben Nickel auch Kupfer und Chrom, sowie neben Nickel und Kobalt auch Eisen enthalten. Das Ruthenium wird auf den Katalysator in Form einer Lösung eines Rutheniumhalogenids aufgebracht. Der Katalysator wird beispielsweise zur Aminierung von Monoethanolamin mit Ammoniak verwendet, wobei die Umsetzung in einem Autoklaven durchgeführt wird.

Aus der EP-A-0 254 335 ist ein Verfahren zur Herstellung eines Hydrierungs- und/oder Dehydrierungskatalysators bekannt, wobei ca. 10 Gew.-% Nickel oder Kobalt und ca. 0,5 Gew.-% Ruthenium, bezogen auf das Gesamtgewicht des Katalysators, auf einen Aluminiumoxidträger aufgebracht werden. Der Katalysator wird durch Imprägnieren des Trägers mit einer Nickel- oder Kobaltnitratlösung, anschließender Tränkung mit wäßriger Salzsäure und anschließender Tränkung mit einer Lösung von Rutheniumnitrosylnitrat hergestellt. Zur Beschichtung des Trägers wird kein Rutheniumhalogenid verwendet. Der Katalysator wird zur Umsetzung von Monoethanolamin mit Ammoniak in einem Autoklaven eingesetzt.

Die bekannten Katalysatoren weisen große Gehalte an Nickel und/oder Kobalt auf. Zudem ist insbesondere bei den in EP-A-0 254 335 beschriebenen Katalysatoren die Haltbarkeit unzureichend. Die bei der Umsetzung von Monoethanolamin mit Ammoniak erhaltenen Selektivitäten in bezug auf Ethylendiamin sind bei kontinuierlicher Verfahrensführung unzureichend.

Aufgabe der vorliegenden Erfindung ist die Bereitstellung von Aminierungskatalysatoren, die die Nachteile der bekannten Katalysatoren vermeiden und insbesondere einen geringen Gehalt an Nickel und/oder Kobalt aufweisen bei gleichzeitig guter Haltbarkeit und hoher Selektivität in bezug auf Ethylendiamin bei der Aminierung von Monoethanolamin mit Ammoniak.

Die Aufgabe wird durch Bereitstellung eines Katalysators gelöst, umfassend bezogen auf das Gesamtgewicht des Katalysators

| | |
|---|---|
| 0,4 - 2 Gew.-% | Kobalt, Nickel oder deren Gemisch, |
| 0,1 - 10 Gew.-% | Ruthenium, |
| 0 - 10 Gew.-% und | Kupfer |
| 0 - 5 Gew.-% | Promotoren aus der Gruppe Eisen, Rhodium, Palladium, Platin, Iridium, Osmium, Silber, Gold, Chrom, Molybdän, Wolfram, Rhenium, Zink, Cadmium, Blei, Mangan, Zinn, Lithium, Natrium, Kalium, Rubidium, Cäsium, Phosphor, Arsen, Antimon, Wismut, Tellur, Thallium oder Gemischen davon |

auf einem porösen Metalloxidträger.

Als Träger des Katalysators wird ein poröses Metalloxid eingesetzt, das ausgewählt ist aus Aluminiumoxid, Alumosilicaten, Titandioxid, Zirkoniumdioxid, Magnesiumoxid oder Gemischen davon. Vorzugsweise werden Träger verwendet, die Aluminiumoxid enthalten und besonders bevorzugt aus Aluminiumoxid bestehen.

Auf den Katalysatorträger sind 0,4 - 2 Gew.-% Kobalt, Nickel oder deren Gemisch aufgebracht. Die Gewichtsangaben beziehen sich dabei auf das Gesamtgewicht des Katalysators, sofern nichts anderes angegeben ist. Vorzugsweise sind auf den Träger 0,1 - 3, besonders bevorzugt 0,2 - 2, insbesondere 0,4 - 1 Gew.-% Kobalt aufgebracht. Ebenso sind vorzugsweise 0,1 - 3, besonders bevorzugt 0,2 - 2, insbesondere 0,4 - 1 Gew.-% Nickel aufgebracht.

Der Katalysator enthält außerdem 0,1 - 10, vorzugweise 0,3 - 4, besonders bevorzugt 0,5 - 2,5 Gew.-% Ruthenium auf dem Träger.

Zudem kann Kupfer auf den Träger aufgebracht sein. Der Gehalt an Kupfer beträgt dabei 0 - 10, vorzugsweise 0,1 - 7, besonders bevorzugt 0,5 - 4 Gew.-%.

Zudem können auf dem Träger 0 - 5 Gew.-% Promotoren vorliegen. Sie sind ausgewählt aus der Gruppe Eisen, Rhodium, Palladium, Platin, Iridium, Osmium, Silber, Gold, Chrom, Molybdän, Wolfram, Rhenium, Zink, Cadmium, Blei, Mangan, Zinn, Lithium, Natrium, Kalium, Rubidium, Cäsium, Phosphor, Arsen, Antimon, Wismut, Tellur, Thallium oder Gemischen davon.

Besonders bevorzugt enthält der Katalysator 0,4 - 1 Gew.-% Kobalt, 0,4 - 1 Gew.-% Nickel, 0,3 - 4 Gew.-% Ruthenium und 0,5 - 4 Gew.-% Kupfer. Insbesondere beträgt der Gehalt an Nickel 0,7 - 0,9 Gew.-%, Kobalt 0,7 - 0,9 Gew.-%, Kupfer 1,3 - 1,9 Gew.-% und Ruthenium 0,5 - 2,5 Gew.-%.

Durch zusätzliche Dotierung der Katalysatorträger mit den vorstehend aufgeführten Promotoren kann die Selektivität des Katalysators gesteuert werden. Vorzugsweise beträgt der Gehalt an Promotor 0,001 - 5, insbesondere 0,01 - 3 Gew.-%.

Die Katalysatoren werden hergestellt durch
(a) Beschichten des Trägers mit den Metallen, Promotoren oder Verbindungen davon,
(b) Trocknen und Calcinieren des imprägnierten Trägers und
(c) Reduzieren des calcinierten Trägers im Wasserstoffstrom.

Im folgenden werden die Metalle und die Promotoren abgehandelt und gemeinsam als Metalle bezeichnet.

Das Beschichten des Trägers mit den Metallen oder Verbindungen der Metalle im Schritt (a) kann durch beliebige geeignete Verfahren erfolgen.

Beispielsweise kann der Träger mit einer Lösung der Metallverbindungen getränkt werden. Er kann auch durch Aufsprühen der Lösungen oder durch gemeinsames Verkneten oder durch Ausfällung der Metalle oder Metallverbindungen auf den Träger imprägniert werden.

Die Metalle, mit denen der Träger imprägniert wird, werden dabei vorzugsweise in Form einer Lösung aus den Salzen der Metalle eingesetzt. Beispielsweise können die Nitrate, Halogenide, insbesondere Chloride, Formiate, Oxalate oder Ammoniakate, vorzugsweise die Oxalate und Nitrate, besonders bevorzugt die Nitrate eingesetzt werden. Gemäß einer Ausführungsform der Erfindung wird Ruthenium in Form einer halogenidhaltigen Lösung eingesetzt. Die Lösung kann dabei aus den Halogeniden des Rutheniums, insbesondere aus den Chloriden hergestellt werden. Vorzugsweise wird Ruthenium in Form einer halogenidfreien Lösung eingesetzt, vorzugsweise in Form einer Lösung eines Salzes, wie es vorstehend beschrieben ist. Besonders bevorzugt sind Oxalate und Nitrate, insbesondere Nitrate von Ruthenium.

Gemäß einer besonders bevorzugten Ausführungsform der Erfindung wird der Träger nicht mit Halogenverbindungen imprägniert. Dabei wird der Träger insbesondere nicht mit Metallhalogeniden oder Lösungen der Metallhalogenide oder mit einer anderen halogenidhaltigen Lösung, wie wäßriger Salzsäurelösung, imprägniert.

Die Erfindung betrifft auch einen wie vorstehend beschriebenen Katalysator, der herstellbar ist durch
(a) Imprägnieren des Trägers mit den Metallen, Promotoren oder Verbindungen davon,
(b) Trocknen und Calcinieren des imprägnierten Trägers und
(c) Reduzieren des calcinierten Trägers in Wasserstoffstrom,
wobei der Träger nicht mit Halogenverbindungen imprägniert wird.

Insbesondere das Tränken des Trägers mit einer wäßrigen Salzsäurelösung, wie es in EP-A-0 254 335 beschrieben ist, kann zu verschlechterten mechanischen Eigenschaften des Katalysators führen, da die oxidischen Träger von der Säure angegriffen werden können. Hierdurch kann die mechanische Stabiltät des Katalysators so vermindert werden, daß die Standzeit des Katalysators im Betrieb aufgrund von Katalysatorzerfall sinkt. Auf dem Katalysatorträger vorliegende Chloride können zudem sowohl bei der Katalysatorherstellung als auch bei Umsetzungen mit dem Katalysator zu Korrosionsproblemen führen, wenn beispielsweise Salzsäure aus dem Katalysator freigesetzt wird. Deshalb ist der Katalysator vorzugsweise halogenidfrei, und es werden bei der Herstellung des Katalysators keine Halogenide, insbesondere Chloride verwendet. Insbesondere wird der Katalysatorträger vorzugsweise nicht mit Halogenverbindungen imprägniert.

Die Imprägnierung des Katalysators mit den Metallen oder Metallsalzlösungen kann in beliebiger Reihenfolge erfolgen. Beispielsweise kann der Katalysatorträger mit einer Lösung, die sämtliche Metallsalze enthält, imprägniert werden. Der Träger kann auch mit mehreren Lösungen, die Salze eines oder mehrerer der verwendeten Metalle enthalten, nacheinander imprägniert werden. Dabei können alle oder einzelne Imprägnierungsschritte mehrfach ausgeführt werden, wobei die Reihenfolge der Imprägnierungen verändert werden kann. Beim mehrfachen Imprägnieren, insbesondere beim mehrfachen Aufsprühen der Lösungen oder bei mehrfacher Tränkung des Trägers mit den Lösungen kann die Konzentration der Metallsalze in den Lösungen gering gehalten werden.

Die Konzentration in den Lösungen beziehungsweise der Lösung kann auch so eingestellt werden, daß durch einfaches Aufbringen oder Tränken die gewünschte Metallmenge auf dem Träger vorliegt. Nach dem Imprägnieren des Trägers mit den Metallen oder Verbindungen der Metalle wird der imprägnierte Träger vorzugsweise bei Temperaturen von 80 - 150°C, besonders bevorzugt von 80 - 120°C getrocknet. Sodann wird der imprägnierte Träger bei Temperaturen von 150 - 500°C, vorzugsweise 300 - 500°C calciniert. Anschließend wird der imprägnierte und calcinierte Träger im Wasserstoffstrom bei einer Temperatur von 150 - 500°C, vorzugsweise 200 - 400°C reduziert. Dazu wird der Träger nach dem Calcinieren vorzugsweise zunächst abgekühlt. Der Wasserstoffstrom kann als reiner Wasserstoffstrom oder als verdünnter Wasserstoffstrom, beispielsweise in einem Inertgas wie Stickstoff eingesetzt werden.

Die Reduktion, die auch als Aktivierung bezeichnet werden kann, kann direkt im Reaktor durchgeführt werden, der auch für die nachfolgende Synthese eingesetzt wird. Wird die Reduktion der Katalysatoren in einem separaten Reaktor durchgeführt, so werden die Katalysatoren vor dem Ausbau aus dem Reaktor vorzugsweise bei Temperaturen von 10 - 60, insbesondere 20 - 40°C mit einem Gasgemisch, das freien Sauerstoff enthält, oberflächlich passiviert. Die so passivierten Katalysatoren können nach dem Einbau in den zur Synthese vorgesehenen Reaktor in einem Wasserstoffstrom, vorzugsweise Stickstoff-/Wasserstoffstrom bei einer Temperatur von 150 - 200°C, vorzugsweise 170 - 190°C aktiviert werden. Sie können auch ohne weitere Aktivierung eingesetzt werden.

Die erfindungsgemäßen Katalysatoren können in jeder geeigneten Form eingesetzt werden, beispielsweise als Formkörper, wie Stränge oder Pellets, oder in Pulverform. Dabei können vorgeformte Träger mit den Metallen oder Metallverbindungen imprägniert werden, oder imprägnierte Träger in die gewünschte Form gebracht werden. Bei dem gemeinsamen Verkneten oder der gemeinsamen Fällung von Trägermaterial und Metallen oder Metallverbindungen werden die resultierenden Massen in der Regel anschließend verformt. Insbesondere bei kontinuierlicher Verfahrensführung werden die Katalysatoren in Form von Formkörpern verwendet.

Die erfindungsgemäßen Katalysatoren können in einer Vielzahl von Umsetzungen eingesetzt werden. Vorzugsweise werden sie erfindungsgemäß in Hydrierungsreaktionen, Dehydrierungsreaktionen oder Hydrierungs-/Dehydrierungsreaktionen eingesetzt. Insbesondere werden die Katalysatoren zur Aminierung von Alkylenoxiden, Alkoholen, Aldehyden oder Ketonen mit Ammoniak oder primären oder sekundären Aminen eingesetzt. Besonders bevorzugt werden die Katalysatoren zur Aminierung von Alkoholen, speziell bei der Umsetzung von Monoethanolamin mit Ammoniak zu Ethylendiamin eingesetzt. Dabei sind die erfindungsgemäßen Katalysatoren über eine lange Zeit mechanisch stabil und zeigen keinen Abfall der Aktivität. Sie zeigen bei gleicher Aktivität im Vergleich zu bekannten Katalysatoren mit hohem Gehalt an Nickel und Kobalt eine höhere Selektivität in bezug auf die Bildung primärer Aminierungsprodukte bei der Aminierung mit Ammoniak. Sie weisen zudem eine wesentlich verbesserte Standzeit auf. Durch den geringen Gehalt an Nickel und Kobalt sind sie wirtschaftlicher herstellbar.

Die Erfindung betrifft auch ein Verfahren zur Herstellung von Aminierungsprodukten durch Umsetzung von Alkylenoxiden, Alkoholen, Aldehyden oder Ketonen mit Ammoniak, primären oder sekundären Aminen in Gegenwart von freiem Wasserstoff und in Gegenwart eines wie vorstehend beschriebenen Katalysators.

Die Aminierung erfolgt dabei durch Umsetzung von Ammoniak oder einer primären oder sekundären Aminogruppe mit einer Hydroxylgruppe, Aldehyd- oder Ketogruppe oder einer Alkylenoxidgruppe, wobei Aldehyd-, Keto- oder Alkylenoxidgruppen reduktiv aminiert werden beziehungsweise Hydroxylgruppen durch Aminogruppen ersetzt werden. Dabei können die Aminogruppen und zu aminierenden Gruppen in unterschiedlichen Molekülen oder auch im gleichen Molekül vorliegen. Liegen die miteinander umzusetzenden Gruppen im gleichen Molekül vor, so können cyclische Verbindungen resultieren. Die Verbindungen, in denen Aminogruppen und zu aminierende Gruppen vorliegen, können zudem entweder als Aminkomponente oder als zu aminierende Verbindung wirken.

Beispielsweise können bei der Aminierung von Monoethanolamin (MEA) mit Ammoniak unter anderem Ethylendiamin (EDA), Aminoethylethanolamin (AEEA), Diethylentriamin (DETA) und Piperazin erhalten werden.

Es können auch Verbindungen eingesetzt werden, die zwei oder mehrere der Hydroxylgruppen, Aldehyd-, Keto- oder Alkylenoxidgruppen aufweisen. Dabei können auch gemischte Gruppen vorliegen. Insbesondere können Diole oder Polyole, insbesondere Ethylenglykole eingesetzt werden. Weiterhin können auch Verbindungen eingesetzt werden, die mehrere primäre oder sekundäre Aminogruppen aufweisen, wie Alkylendiamine, insbesondere Ethylendiamin. Beispielsweise können Ethylenglykole oder Ethanolamine mit oder in Gegenwart von Ammoniak, Ethanolaminen, Ethylendiaminen oder Diethylentriaminen aminiert werden.

Gemäß einer Ausführungsform der Erfindung dient das erfindungsgemäße Verfahren zur Herstellung von Aminen der allgemeinen Formel (I)

R¹R²N-CHR³R⁴ (I)

in der R¹, R², R³ und R⁴ unabhängig Wasserstoff, C₁₋₂₀₀-Alkyl, C₃₋₁₂-Cycloalkyl, durch Amino, C₁₋₂₀-Alkylamino, (Di-C₁₋₂₀-alkyl)amino und/oder Hydroxy substituiertes C₁₋₂₀-Alkyl, C₂₋₃₀-Alkoxyalkyl, R⁵(OCHR⁶CH₂)ₙ, Aryl, C₇₋₂₀-Aralkyl oder C₇₋₂₀-Alkylaryl bedeuten oder R¹ oder R² gemeinsam (CH₂)₁-X-(CH₂)ₘ bilden,
wobei
R⁵ Wasserstoff oder C₁₋₄-Alkyl,
R⁶ Wasserstoff oder Methyl,
X Sauerstoff oder NR⁶ bedeuten,
n eine ganze Zahl von 2 - 30,
l eine ganze Zahl von 2 - 4,
m eine ganze Zahl von 1 - 4 ist,
aus primären oder sekundären Alkoholen, Ketonen, Aldehyden der allgemeinenen Formel (II)

R³R⁴CHOH oder R³R⁴CO (II)

in der R³ und R⁴ die vorstehend angegebene Bedeutung haben, und primären oder sekundären Aminen der allgemeinen Formel (III)

R¹R²NH (III)

in der R¹ und R² die vorstehend angegebene Bedeutung haben.

Die Reste R¹, R², R³, R⁴, insbesondere die Reste R¹ und R² können dabei C₁₋₂₀₀-Alkyl, vorzugsweise C₁₋₈-Alkyl sein, wie Methyl, Ethyl, n-Propyl, Isopropyl, n-Butyl, Isobutyl, sec.-Butyl, tert.-Butyl, n-Pentyl, Isopentyl, sec.-Pentyl, neo-Pentyl, 1,2-Dimethylpropyl, n-Hexyl, Isohexyl, sec.-Hexyl, n-Heptyl, Isoheptyl, n-Octyl, Isooctyl, 2-Ethylhexyl, n-Decyl, 2-n-Propyl-n-heptyl, n-Tridecyl, 2-n-Butyl-n-nonyl, 3-n-Butyl-n-nonyl, besonders bevorzugt Isopropyl, 2-Ethylhexyl, n-Decyl, 2-n-Propyl-n-heptyl, n-Tridecyl, 2-n-Butyl-n-nonyl und 3-n-Butyl-n-nonyl, sowie C₄₀₋₂₀₀-Alkyl, wie Polybutyl, Polyisobutyl, Polypropyl, Polyisopropyl und Polyethyl, besonders bevorzugt Polybutyl und Polyisobutyl.

R¹ und R² können gemeinsam -(CH₂)₁-X-(CH₂)ₘ sein, wobei X Sauerstoff oder N-R⁶ ist mit der nachstehend angegebenen Bedeutung und l eine ganze Zahl von 2 - 4, wie 2, 3 oder 4, bevorzugt 2 oder 3, besonders bevorzugt 2 und m eine ganze Zahl von 1 - 4, wie 1, 2, 3 oder 4, bevorzugt 2, 3 oder 4, besonders bevorzugt 2 oder 3 ist.

R¹, R², R³, R⁴ können C₃₋₁₂-Cycloalkyl, bevorzugt C₃₋₈-Cycloalkyl sein, wie Cyclopropyl, Cyclobutyl, Cyclopentyl, Cyclohexyl, Cycloheptyl, Cyclooctyl, besonders bevorzugt Cyclopentyl, Cyclohexyl und Cyclooctyl.

Sie können Aryl sein, wie Phenyl, 1-Naphthyl, 2-Naphthyl, 1-Anthryl, 2-Anthryl, 9-Anthryl, bevorzugt Phenyl, 1-Naphthyl, 2-Naphthyl, besonders bevorzugt Phenyl.

Sie können C₇₋₂₀-Alkylaryl, bevorzugt C₇₋₁₂-Alkylphenyl sein, wie 2-Methylphenyl, 3-Methylphenyl, 4-Methylphenyl, 2,4-Dimethylphenyl, 2,5-Dimethylphenyl, 2,6-Dimethylphenyl, 3,4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl, 4-n-Propylphenyl.

Sie können C₇₋₂₀-Aralkyl, bevorzugt C₇₋₁₂-Phenylalkyl sein, wie Benzyl, 1-Phenylethyl, 2-Phenylethyl, 1-Phenylpropyl, 2-Phenylpropyl, 3-Phenylpropyl, 1-Phenylbutyl, 2-Phenylbutyl, 3-Phenylbutyl, 4-Phenylbutyl, besonders bevorzugt Benzyl, 1-Phenylethyl und 2-Phenylethyl.

R¹ kann insbesondere ein C₁₋₂₀-Alkyl, bevorzugt C₁₋₈-Alkyl sein, wie Methyl, Ethyl, n-Propyl, Isopropyl, n-Butyl, Isobutyl, 6-Butyl, tert.-Butyl, n-Pentyl, Isopentyl, sec.-Pentyl, neo-Pentyl, 1,2-Dimethylpropyl, n-Hexyl, Isohexyl, sec.-Hexyl, n-Heptyl, Isoheptyl, n-Octyl, Isooctyl, besonders bevorzugt C₁₋₄-Alkyl, wie Methyl, Ethyl, n-Propyl, Isopropyl, n-Butyl, Isobutyl, sec.-Butyl, tert.-Butyl.

R³ und R⁴ können insbesondere C₁₋₂₀-Hydroxyalkyl, bevorzugt C₁₋₈-Hydroxyalkyl sein, besonders bevorzugt C₁₋₄-Hydroxyalkyl, wie Hydroxymethyl, 1-Hydroxyethyl, 2-Hydroxyethyl, 1-Hydroxy-n-propyl, 2-Hydroxy-n-propyl, 3-Hydroxy-n-propyl, 1-Hydroxymethylethyl, durch Amino und/oder Alkylamino und/oder Dialkylamino und/oder Hydroxy substituiertes C₁₋₂₀-Alkyl, bevorzugt C₁₋₈-Alkyl, besonders bevorzugt C₁₋₄-Alkyl, wie N-(Hydroxyethyl)aminoethyl und N-(Aminoethyl)aminoethyl.

Sie können C₂₋₃₀-, bevorzugt C₂₋₂₀-, besonders bevorzugt C₂₋₈-Alkoxyalkyl sein, wie Methoxymethyl, Ethoxymethyl, n-Propoxymethyl, Isopropoxymethyl, n-Butoxymethyl, Isobutoxymethyl, sec.-Butoxymethyl, tert.-Butoxymethyl, 1-Methoxymethyl, 2-Methoxymethyl, besonders bevorzugt C₂₋₄-Alkoxyalkyl, wie Methoxymethyl, Ethoxymethyl, n-Propoxymethyl, Isopropoxymethyl, n-Butoxymethyl, Isobutoxymethyl, sec.-Butoxymethyl, tert.-Butoxymethyl, 1-Methoxyethyl und 2-Methoxyethyl.

Sie können R⁵-(OCHR⁶CH₂)ₙ sein, wobei R⁵ C₁₋₄-Alkyl ist, wie Methyl, Ethyl, n-Propyl, Isopropyl, n-Butyl, Isobutyl, sec.-Butyl, tert.-Butyl, bevorzugt Methyl oder Ethyl, besonders bevorzugt Methyl. R⁶ ist dabei Wasserstoff oder Methyl.

n ist eine ganze Zahl von 2 - 10, bevorzugt von 2 - 8, wie 2, 3, 4, 5, 6, 7 oder 8, besonders bevorzugt 2, 3, 4, 5, 6.

Alle Reste R¹ - R⁶ können auch Wasserstoffatome sein.

Das erfindungsgemäße Verfahren kann diskontinuierlich oder bevorzugt kontinuierlich durchgeführt werden. Bei der kontinuierlichen Durchführung wird dabei vorzugsweise in einem Rohrreaktor in Riesel- oder Sumpffahrweise gearbeitet. Insbesondere bei der Aminierung von Alkoholen wird bei Temperaturen von 120 - 250°C, bevorzugt 150 - 190°C und Drücken von 150 - 300 bar, bevorzugt 180 - 220 bar gearbeitet. Allgemein kann bei Temperaturen von 80 - 250°C und Drücken von 100 - 400 bar gearbeitet werden.

Bei der Umsetzung mit Ammoniak kann Ammoniak als Reagenz und als Lösungsmittel eingesetzt werden. Dabei können pro Mol Alkylenoxid, Alkohol, Aldehyd oder Keton 1 - 20, vorzugsweise 6 - 12 Mol Ammoniak eingesetzt werden. Die Katalysatorbelastung beträgt vorzugsweise 0,05 bis 2,0, besonders bevorzugt 0,1 - 1,0 kg Alkylenoxid, Alkohol, Aldehyd oder Keton pro Liter Katalysator pro Stunde. Die Umsetzung kann auch in Gegenwart von Wasser durchgeführt werden, wobei bis zu 15 Gew.-%, bezogen auf das gesamte Reaktionsgemisch, Wasser zugesetzt werden können.

Die Verwendung der erfindungsgemäßen Katalysatoren im erfindungsgemäßen Verfahren erlaubt insbesondere bei der Umsetzung von Monoethanolamin mit Ammoniak eine hohe Ausbeute an Ethylendiamin, bedingt durch die hohe Selektivität der erfindungsgemäßen Katalysatoren.

Die nach dem erfindungsgemäßen Verfahren erhaltenen Produkte eignen sich u.a. als Zwischenprodukte bei der Herstellung von Kraftstoffadditiven, beispielsweise beschrieben in US 3,275,554, DE-A-21 25 039 oder DE-A-36 11 230. Ebenso können die erfindungsgemäß erhaltenen Verbindungen bei der Herstellung von Tensiden, Arznei- und Pflanzenschutzmitteln, sowie Vulkanisationsbeschleunigern eingesetzt werden. Nachstehend wird die Erfindung zusätzlich anhand von Beispielen erläutert.

### BEISPIEL 1

135 g Al₂O₃-Stränge mit einem Durchmesser von 4 mm (D10-10, hergestellt von der BASF AG, Ludwigshafen) wurden mit 88 ml einer wäßrigen Tränklösung, die 1,4 g NiO, 1,4 g CoO und 2,5 g CuO enthielt, unter mehrmaligem guten Durchmischen beim Raumtemperatur für zwei Stunden stehen gelassen. Der Katalysatorvorläufer wurde für 16 Stunden bei 120°C getrocknet und für vier Stunden bei 400°C calciniert. Sodann wurden die Stränge mit 88 ml einer wäßrigen Rutheniumchloridlösung, die 1,41 g Ruthenium enthielt, getränkt. Nickel, Kobalt und Kupfer wurden dabei in Form der Nitrate eingesetzt.

Anschließend wurde der Katalysatorvorläufer 16 Stunden bei 120°C getrocknet und für vier Stunden bei 400°C calciniert. Nach dem Abkühlen wurden die Stränge in eine Reduktionsapparatur eingebaut und für zwei Stunden mit 20 l N₂/h gespült. Danach wurde mit einer Rate von 2°C/min und 20 l H₂/h Wasserstofffluß auf 300°C aufgeheizt und 20 Stunden bei dieser Temperatur gehalten. Nach Abkühlen im Stickstoffstrom wurde der Katalysator mit einem Luft/Stickstoff-Gemisch passiviert, wobei die Temperaturerhöhung maximal 20°C betragen darf. Der so hergestellte Katalysator enthielt 1 Gew.-% Ruthenium, 0,79 Gew.-% Nickel, 0,79 Gew.-% Kobalt und 1,6 Gew.-% Kupfer auf Aluminiumoxid. Die Ergebnisse bei der Aminierung von Monoethanolamin sind in Tabelle 1 zusammengestellt. Der Katalysator war nach einer Betriebszeit von 143 Stunden noch vollständig erhalten.

### BEISPIEL 2

Der Katalysator wurde analog Beispiel 1 hergestellt. Die Ergebnisse bei der Aminierung von Monoethanolamin mit gegenüber Beispiel 1 vermindertem Umsatz sind in Tabelle 1 zusammengestellt. Der Katalysator war nach einer Betriebszeit von 133 Stunden vollständig erhalten.

### BEISPIEL 3

Der Katalysator wurde analog Beispiel 1 hergestellt. Die Ergebnisse bei der Aminierung von Monoethanolamin mit gegenüber Beispiel 1 vermindertem Umsatz sind in Tabelle 1 zusammengestellt. Der Katalysator war nach einer Betriebszeit von 153 Stunden noch vollständig erhalten.

### BEISPIEL 4

Der Katalysator wurde analog Beispiel 1 hergestellt, jedoch wurden 500 g der Al₂O₃-Stränge mit 311 ml einer wäßrigen Tränklösung, die 5,3 g NiO, 5,3 g CoO und 10,6 g CuO in Form der Nitrate enthielt, getränkt, sodann getrocknet und calciniert. Dann wurden die Stränge mit 248 ml einer wäßrigen Rutheniumnitratlösung, die 10,6 g Ruthenium enthielt, getränkt. Das Trocknen und Calcinieren sowie die Reduktion erfolgte wie in Beispiel 1 angegeben. Der so hergestellte Katalysator enthielt 2 Gew.-% Ruthenium, 0,79 Gew.-% Nickel, 0,79 Gew.-% Kobalt und 1,6 Gew.-% Kupfer auf Aluminiumoxid. Die Ergebnisse bei der Aminierung von Monoethanolamin sind in Tabelle 1 zusammengestellt. Der Katalysator war nach einer Betriebszeit von 141 Stunden noch vollständig erhalten.

In den nachstehenden Beispielen 5 bis 7 wurde der Umsatz variiert.

### BEISPIEL 5

Der Katalysator wurde analog Beispiel 4 hergestellt. Die Ergebnisse bei der Aminierung von Monoethanolamin sind in Tabelle 1 zusammengestellt. Der Katalysator war nach einer Betriebszeit von 283 Stunden noch vollständig erhalten.

### BEISPIEL 6

Der Katalysator wurde analog Beispiel 4 hergestellt. Die Ergebnisse bei der Aminierung von Monoethanolamin sind in Tabelle 1 zusammengestellt. Der Katalysator war nach einer Betriebszeit von 141 Stunden noch vollständig erhalten.

### BEISPIEL 7

Der Katalysator wurde analog Beispiel 4 hergestellt. Die Ergebnisse bei der Aminierung von Monoethanolamin sind in Tabelle 1 zusammengestellt. Der Katalysator war nach einer Betriebszeit von 251 Stunden noch vollständig erhalten.

### Vergleichsbeispiel V1

Der Katalysator wurde nach dem in EP-A-0 254 335, Beispiel 1 angegebenen Verfahren hergestellt. Der so erhaltene Katalysator enthielt 10 Gew.-% Nickel und 0,5 Gew.-% Ruthenium auf Aluminiumoxid (D10-10 der BASF AG, Ludwigshafen). Die Ergebnisse bei der Aminierung von Monoethanolamin sind in Tabelle 1 zusammengestellt. Der Katalysator war nach einer Betriebszeit von 48 Stunden vollständig zerfallen.

### Vergleichsbeispiel V2

Der Katalysator wurde nach dem in EP-A-0 254 335, Beispiel 13 angegebenen Verfahren hergestellt. Der so erhaltene Katalysator enthielt 10 Gew.-% Kobalt, 0,5 Gew.-% Ruthenium auf Aluminiumoxid (D10-10 der BASF AG, Ludwigshafen). Die Ergebnisse bei der Aminierung von Monoethanolamin sind in Tabelle 1 zusammengestellt. Der Katalysator war nach einer Betriebszeit von 48 Stunden vollständig zerfallen.

### Aminierung:

Die Aminierung von Monoethanolamin in Gegenwart der Katalysatoren der Beispiele 1 - 7 und Vergleichsbeispiele V1 und V2 wurde wie folgt durchgeführt: ein 100 ml fassender Rohrreaktor mit einer Länge von 55 cm und einem Innendurchmesser von 1,5 cm wurde mit 50 g passiviertem Katalysator befüllt und der Katalysator bei 180°C zunächst mit einem Gemisch aus 20 Vol.-% Wasserstoff / 80 Vol.-% Stickstoff und anschließend mit 100 Vol.-% Wasserstoff aktiviert. Nach Einstellung der Reaktionstemperatur auf 175 - 195°C in Abhängigkeit von der Aktivität des Katalysators wurde der Reaktor mit 10 - 30 g/h Monoethanolamin, 20 - 70 g/h Ammoniak und 3 - 10 Nl/h Wasserstoff beschickt. Durch gaschromatographische Analyse des Austrags wurden der Umsatz und die Selektivität bezüglich der Komponenten Ethylendiamin (EDA), Aminoethylethanolamin (AEEA), Diethylentriamin (DETA) und Piperazin bestimmt. Die Katalysatorbelastung ist in bezug auf Monoethanolamin (MEA) angegeben. Die Ergebnisse sind in der nachstehenden Tabelle 1 zusammengefaßt.

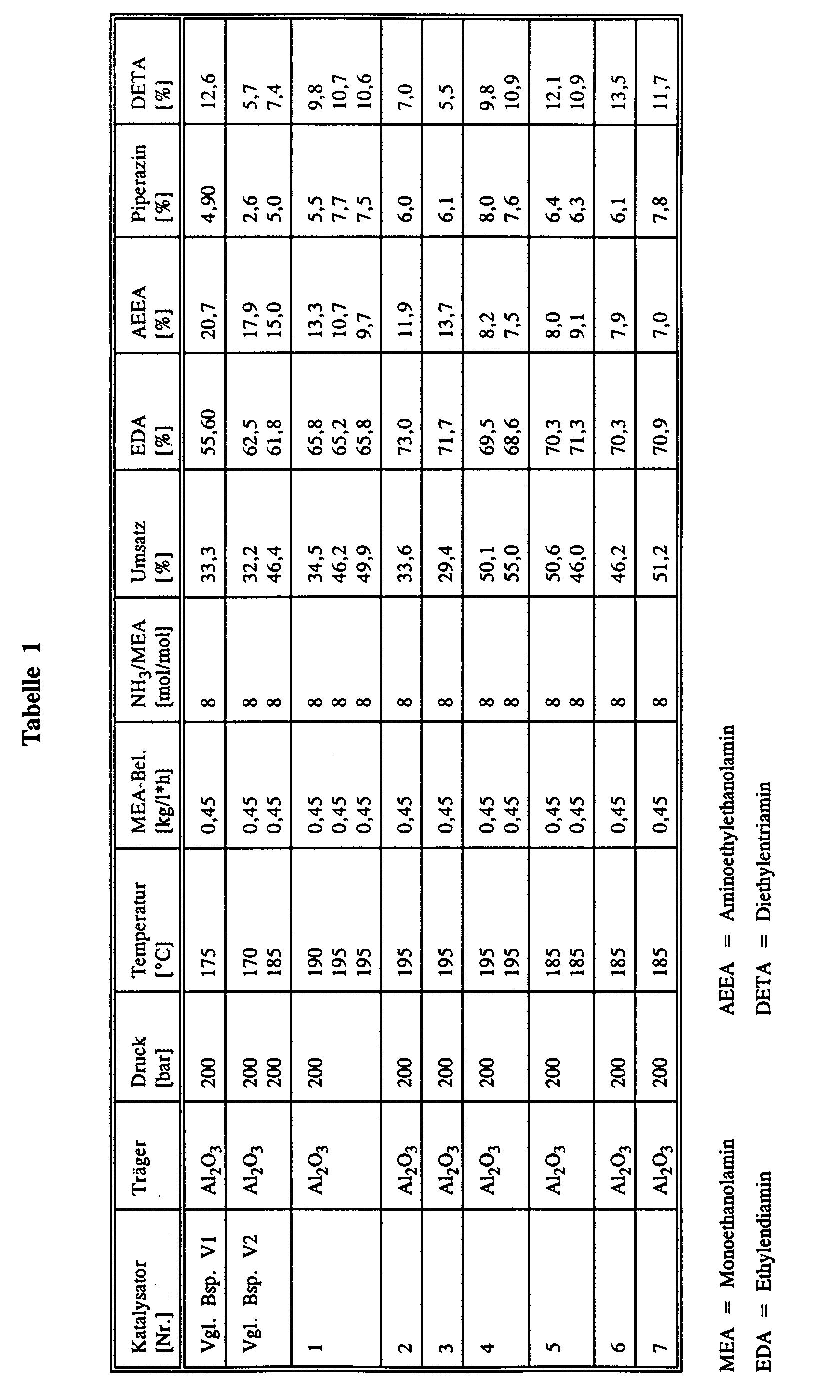

Aus den Ergebnissen der Tabelle 1 geht hervor, daß bei Verwendung der erfindungsgemäßen Katalysatoren die Selektivität in bezug auf Ethylendiamin deutlich höher ist als bei Verwendung der bekannten Katalysatoren. Zudem ist die Haltbarkeit der erfindungsgemäßen Katalysatoren wesentlich höher als die Haltbarkeit der Vergleichskatalysatoren.

## Patentansprüche

1. Katalysator, umfassend bezogen auf das Gesamtgewicht des Katalysators
| | |
|---|---|
| 0,4 - 2 Gew.-% | Kobalt, Nickel oder deren Gemisch, |
| 0,1 - 10 Gew.-% | Ruthenium, |
| 0 - 10 Gew.-% und | Kupfer |
| 0 - 5 Gew.-% | Promotoren aus der Gruppe Eisen, Rhodium, Palladium, Platin, Iridium, Osmium, Silber, Gold, Chrom, Molybdän, Wolfram, Rhenium, Zink, Cadmium, Blei, Mangan, Zinn, Lithium, Natrium, Kalium, Rubidium, Cäsium, Phosphor, Arsen, Antimon, Wismut, Tellur, Thallium oder Gemischen davon |
auf einem porösen Metalloxidträger.

2. Katalysator nach Anspruch 1, umfassend 0,4 - 1 Gew.-% Kobalt und 0,4 - 1 Gew. - % Nickel.

3. Katalysator nach Anspruch 1 oder 2, wobei der poröse Metalloxidträger ausgewählt ist aus Aluminiumoxid, Siliciumdioxid, Alumosilicaten, Titandioxid, Zirkoniumdioxid, Magnesiumoxid oder Gemischen davon.

4. Katalysator nach einem der Ansprüche 1 bis 3, herstellbar durch
(a) Imprägnieren des Trägers mit den Metallen, Promotoren oder Verbindungen davon,
(b) Trocknen und Calcinieren des imprägnieren Trägers und
(c) Reduzieren des calcinierten Trägers im Wasserstoffstrom,
wobei der Träger nicht mit Halogenverbindungen imprägniert wird.

5. Verfahren zur Herstellung eines Katalysators nach einem der Ansprüche 1 bis 4 durch
(a) Imprägnieren des Trägers mit den Metallen, Promotoren oder Verbindungen davon,
(b) Trocknen und Calcinieren des imprägnierten Trägers und
(c) Reduzieren des calcinierten Trägers in Wasserstoffstrom.

6. Verwendung des Katalysators nach einem der Ansprüche 1 bis 4 in Hydrierungsreaktionen, Dehydrierungsreaktionen oder Hydrierungs-/Dehydrierungsreaktionen.

7. Verwendung nach Anspruch 6, wobei die Hydrierungs-/Dehydrierungsreaktion eine Aminierung von Alkylenoxiden, Alkoholen, Aldehyden oder Ketonen mit Ammoniak, primären oder sekundären Aminen ist.

8. Verfahren zur Herstellung von Aminierungsprodukten durch Umsetzung von Alkylenoxiden, Alkoholen, Aldehyden oder Ketonen mit Ammoniak, primären oder sekundären Aminen in Gegenwart von freiem Wasserstoff und in Gegenwart eines Katalysators nach einem der Ansprüche 1 bis 4.

9. Verfahren nach Anspruch 8, wobei Ethylenglykole oder Ethanolamine mit oder in Gegenwart von Ammoniak, Ethanolaminen, Ethylendiaminen oder Diethylentriaminen aminiert werden.

10. Verfahren nach Anspruch 8 oder 9, das kontinuierlich ausgeführt wird.

## Claims

1. A catalyst comprising, based on the total weight of the catalyst,
| | |
|---|---|
| 0.4 - 2% by weight | of cobalt, nickel or a mixture thereof, |
| 0.1 - 10% by weight | of ruthenium, |
| 0 - 10% by weight and | of copper |
| 0 - 5% by weight | of promoters selected from the group consisting of iron, rhodium, palladium, platinum, iridium, osmium, silver, gold, chromium, molybdenum, tungsten, rhenium, zinc, cadmium, lead, manganese, tin, lithium, sodium, potassium, rubidium, cesium, phosphorus, arsenic, antimony, bismuth, tellurium, thallium or mixtures thereof |
on a porous metal oxide carrier.

2. A catalyst as claimed in claim 1, comprising 0.4 - 1% by weight of cobalt and 0.4 - 1% by weight of nickel.

3. A catalyst as claimed in claim 1 or 2, wherein the porous metal oxide carrier is selected from alumina, silica, aluminosilicates, titanium dioxide, zirconium dioxide, magnesium oxide and mixtures thereof.

4. A catalyst as claimed in any of claims 1 to 3, which can be prepared by
(a) impregnating the carrier with the metals, promoters or compounds thereof,
(b) drying and calcining the impregnated carrier and
(c) reducing the calcined carrier in a stream of hydrogen,
the carrier not being impregnated with halogen compounds.

5. A process for the preparation of a catalyst as claimed in any of claims 1 to 4 by
(a) impregnating the carrier with the metals, promoters or compounds thereof,
(b) drying and calcining the impregnated carrier and
(c) reducing the calcined carrier in a stream of hydrogen.

6. The use of the catalyst as claimed in any of claims 1 to 4, in hydrogenation reactions, dehydrogenation reactions or hydrogenation/dehydrogenation reactions.

7. The use as claimed in claim 6, wherein the hydrogenation/dehydrogenation reaction is an amination of alkylene oxides, alcohols, aldehydes or ketones with ammonia or primary or secondary amines.

8. A process for the preparation of an amination product by reacting an alkylene oxide, alcohol, aldehyde or ketone with ammonia or a primary or secondary amine in the presence of free hydrogen and in the presence of a catalyst as claimed in any of claims 1 to 4.

9. A process as claimed in claim 8, wherein an ethylene glycol or an ethanolamine is aminated with, or in the presence of, ammonia, an ethanolamine, an ethylenediamine or a diethylenetriamine.

10. A process as claimed in claim 8 or 9, which is carried out continuously.

## Revendications

1. Catalyseur, comprenant, par rapport au poids total du catalyseur,
| | |
|---|---|
| 0,4 à 2% en poids de | cobalt, nickel ou leurs mélanges |
| 0,1 à 10% en poids de | ruthénium, |
| 0 à 10% en poids de et | cuivre |
| 0 à 5% en poids de | promoteurs choisis dans le groupe formé par le fer, le rhodium, le palladium, le platine, l'iridium, l'osmium, l'argent, l'or, le chrome, le molybdène, le tungstène, le rhénium, le zinc, le cadmium, le plomb, le manganèse, l'étain, le lithium, le sodium, le potassium, le rubidium, le césium, le phosphore, l'arsenic, l'antimoine, le bismuth, le tellure, le thallium ou leurs mélanges |
sur un support poreux à base d'oxyde métallique.

2. Catalyseur selon la revendication 1, comprenant 0,4 à 1% en poids de cobalt et 0,4 à 1% en poids de nickel.

3. Catalyseur selon la revendication 1 ou 2, dans lequel le support poreux à base d'oxyde métallique est choisi parmi l'oxyde d'aluminium, le dioxyde de silicium, les aluminosilicates, le dioxyde de titane, le dioxyde de zirconium, l'oxyde de magnésium ou des mélanges de ceux-ci.

4. Catalyseur selon l'une quelconque des revendications 1 à 3, pouvant être préparé
(a) par imprégnation du support avec lesdits métaux, promoteurs ou des composés de ceux-ci,
(b) par séchage et calcination du support imprégné et
(c) par réduction du support calciné dans un courant d'hydrogène,
le support n'étant pas imprégné avec des composés halogénés.

5. Procédé de préparation d'un catalyseur selon l'une quelconque des revendications 1 à 4, par
(a) imprégnation du support avec les métaux, promoteurs ou composés de ceux-ci,
(b) séchage et calcination du support imprégné et
(c) réduction du support calciné dans un courant d'hydrogène.

6. Utilisation du catalyseur selon l'une quelconque des revendications 1 à 4 dans les réactions d'hydrogénation, les réactions de déshydrogénation ou les réactions d'hydrogénation/déshydrogénation.

7. Utilisation selon la revendication 6, dans laquelle la réaction d'hydrogénation/déshydrogénation est une amination d'oxydes d'alkylène, d'alcools, d'aldéhydes ou de cétones avec de l'ammoniac, des amines primaires ou secondaires.

8. Procédé de préparation de produits d'amination par réaction d'oxydes d'alkylène, d'alcools, d'aldéhydes ou de cétones avec de l'ammoniac, des amines primaires ou secondaires en présence d'hydrogène libre et en présence d'un catalyseur selon l'une quelconque des revendications 1 à 4.

9. Procédé selon la revendication 8, dans lequel des éthylèneglycols ou des éthanolamines sont aminés avec de l'ammoniac, des éthanolamines, des éthylène-diamines ou des diéthylène-triamines ou en présence de ces derniers.

10. Procédé selon la revendication 8 ou 9, qui est réalisé en continu.
